# EUROPEAN PATENT APPLICATION

(11) **EP 4 603 819 A1**
(43) Date of publication of application: **20.08.2025**
(21) Application number: 24157341.9
(22) Date of filing: 13.02.2024
(51) Int. Cl.: G01N 1/36, G01N 33/58, G01N 1/30

(54) **ITERATIVE EXPANSION FOR HIGH-FIDELITY TISSUE PRESERVATION**

(71) Applicant: IST Austria - Institute of Science and Technology Austria, 3400 Klosterneuburg (AT)
(72) Inventor: DANZL, Johann Georg, 3400 Klosterneuburg (AT); TAVAKOLI, Mojtaba, 3400 Klosterneuburg (AT)
(74) Representative: Grund, Martin

(57) **Abstract**

The herein disclosed invention relates to a method of preparing a tissue sample for microscopic analysis comprising an iterative expansion scheme. The method comprises use of three different independent and non-crosslinked hydrogels. Moreover, the invention relates to hydrogels prepared according to the disclosed methods and their use in light microscopy.

## Description

### FIELD OF THE INVENTION

The present invention lies in the field of sample preparation for light microscopy combined with hydrogel chemistry.

### BACKGROUND OF THE INVENTION

Microscopy has facilitated the discovery of many biological insights by optically magnifying images of structures in living and fixed cells and tissues. The present invention is based on the fact that physical magnification of the specimen itself is also possible using hydrogel chemistry.

Expansion microscopy (ExM) uses such hydrogel chemistry in order to physically magnify biological specimens through a chemical process, thus enabling nanoimaging on conventional microscopes. The premise is to introduce a polymer network into cellular or tissue samples, and then physically expand that polymer network using water to increase the size of the biological structures. Among other benefits, ExM allows those small structures to be imaged with a wider range of microscopy techniques. Standard ExM protocols comprise embedding a specimen in a hydrogel solution to infuse the specimen with acrylate monomers, polymerization of the acrylate monomers into a swellable polyacrylate hydrogel network and isotropic expansion of the hydrogel upon dialysis with an excess of water (see e.g. K. Chung et al., Nature, vol. 497:332-337 (2013); Chen et al., Science, vol. 347(6221):543-548 (2015); Ku et al., Nature Biotechnology, vol. 34(9):973-981 (2016); Tillberg et al., Nature Biotechnology, vol. 34:987-993 (2016)). Although expanding the hydrogel-embedded specimen to about 4.5-fold was possible, resolution was still not allowing for detailed structural analysis.

It has been found that gel stability and optical resolution can yet be improved with an approach named iterative expansion microscopy (iExM). Iterative expansion microscopy (iExM) is a strategy that achieves high resolution expansion microscopy by expanding samples multiple times (see e.g., Chang et al., Nature Methods, vol. 14(6):593-599 (2017)). In contrast to the conventional expansion microscopy protocols, this strategy includes a second swellable hydrogel gel that expands the specimen further and thus improves resolution. In brief, in the iterative protocol, the information from the first expanded gel is transferred to a second gel, the first hydrogel and a re-embedding hydrogels are is then disrupted, and the second gel is expanded.

However, despite these advances, tracing of very fine and small objects such as neuronal structures and dense brain circuit reconstruction at single-synapse accuracy with conventional light microscopy have been out of reach. Thus, a yet improved protocol of the sample preparation is highly needed in order to be able to also densely reconstruct brain circuitry with light microscopy at synaptic levels.

The present disclosure provides such an improved method in form of a specifically developed high-fidelity iterative hydrogel expansion strategy.

### SUMMARY OF THE INVENTION

As used herein and in the appended claims, the singular forms "a", "an", and "the" are defined to mean "one or more" and include the plural unless the context clearly dictates otherwise. It is further noted that the aspects of the invention can be drafted to exclude any optional element.

The present invention provides a method of preparing a tissue sample for microscopic analysis, hydrogels prepared according to the provided method and uses of the hydrogel.

In one aspect, the invention relates to a method of preparing a tissue sample for microscopic analysis comprising an iterative expansion scheme comprising providing a tissue sample of interest, treating the tissue sample with an epoxide, incubating the tissue sample in a solution for a first expandable hydrogel and subsequently allowing the solution to gel resulting in a hydrogel-embedded tissue sample, denaturing or digesting the hydrogel-embedded tissue sample, expanding the hydrogel-embedded tissue sample by placing the tissue sample in water, neutralizing the hydrogel-embedded tissue sample, incubating the hydrogel-embedded tissue sample in a solution for a stabilizing hydrogel and subsequently allowing the solution to gel to stabilize the hydrogel-embedded tissue sample, neutralizing the hydrogel-embedded tissue sample, incubating the hydrogel-embedded tissue sample in a solution for a second expandable hydrogel and subsequently allowing the solution to gel, and expanding the hydrogel-embedded tissue sample by placing the hydrogel-embedded tissue sample in pure water,
wherein the solution for the first expandable hydrogel comprises
   5-15% (w/v) acrylamide
   7-15% (w/v) sodium acrylate
   0.05-0.1% (w/v) N,N'-methylenebisacrylamide
   0.05-0.2% (w/v) of a polymerization initiator
   0.05-0.2% (w/v) of a polymerization accelerator
   0.0005-0.002% (w/v) TEMPO
wherein the solution for the stabilizing hydrogel comprises
   5-15% (w/v) acrylamide
   0.015-0.05% (w/v) N,N'-methylenebisacrylamide
   0.025-0.075% (w/v) polymerization initiator
   0.025-0.075% (w/v) polymerization accelerator, and
wherein the solution for the second expandable hydrogel comprises
   5-15% (w/v) acrylamide
   12.5-22.5% (w/v) sodium acrylate
   0.015-0.05% (w/v) N,N'-methylenebisacrylamide
   0.025-0.075% (w/v) polymerization initiator
   0.025-0.075% (w/v) polymerization accelerator,
optionally, wherein a molecule-specific labeling of one or more targets of interest is performed.

In an embodiment, the polymerization initiator is APS. In another embodiment, the polymerization accelerator is TEMED. In yet another embodiment, the polymerization initiator is APS and the polymerization accelerator is TEMED. Alternatively, the polymerization initiator and the polymerization accelerator may be replaced by either VA044 or VA050.

In an embodiment, the method of preparing a tissue sample for microscopic analysis thus comprises an iterative expansion scheme comprising providing a tissue sample of interest, treating the tissue sample with an epoxide, incubating the tissue sample in a solution for a first expandable hydrogel and subsequently allowing the solution to gel resulting in a hydrogel-embedded tissue sample, denaturing or digesting the hydrogel-embedded tissue sample, expanding the hydrogel-embedded tissue sample by placing the tissue sample in water, neutralizing the hydrogel-embedded tissue sample, incubating the hydrogel-embedded tissue sample in a solution for a stabilizing hydrogel and subsequently allowing the solution to gel to stabilize the hydrogel-embedded tissue sample, neutralizing the hydrogel-embedded tissue sample, incubating the hydrogel-embedded tissue sample in a solution for a second expandable hydrogel and subsequently allowing the hydrogel solution to gel, and expanding the hydrogel-embedded tissue sample by placing the hydrogel-embedded tissue sample in pure water,
wherein the solution for the first expandable hydrogel comprises
   5-15% (w/v) acrylamide
   7-15% (w/v) sodium acrylate
   0.05-0.1% (w/v) N,N'-methylenebisacrylamide
   0.05-0.2% (w/v) APS
   0.05-0.2% (w/v) TEMED
   0.0005-0.002% (w/v) TEMPO
wherein the solution for the stabilizing hydrogel comprises
   5-15% (w/v) acrylamide
   0.015-0.05% (w/v) N,N'-methylenebisacrylamide
   0.025-0.075% (w/v) APS
   0.025-0.075% (w/v) TEMED, and
wherein the solution for the second expandable hydrogel comprises
   5-15% (w/v) acrylamide
   12.5-22.5% (w/v) sodium acrylate
   0.015-0.05% (w/v) N,N'-methylenebisacrylamide
   0.025-0.075% (w/v) APS
   0.025-0.075% (w/v) TEMED,
optionally, wherein a molecule-specific labeling of one or more targets of interest is performed.

In an embodiment, the epoxide is glycidyl methacrylate (GMA), glycerol triglycidyl ether (TGE) or a mixture thereof. Preferably, the epoxide may be a mixture of GMA and TGE. GMA may be present at a concentration of 0.05-1% (w/v). Moreover, TGE may be present at a concentration of 0.05-1% (w/v). Alternatively, the epoxide may be replaced by or combined with an anchoring compound selected from the group comprising a N-acryloxysuccinimide, acrylic acid N-hydroxysuccinimide ester and Acryloxyl-X. In an embodiment, the epoxide may be replaced by or combined with an anchoring compound selected from the group consisting of a N-acryloxysuccinimide, acrylic acid N-hydroxysuccinimide ester and Acryloxyl-X.

In an embodiment, the molecule-specific labeling comprises immuno-labeling and/or labeling with other affinity probes. Such molecule-specific labeling may comprise incubation with a primary antibody and a fluorophore-conjugated secondary antibody and/or a (DNA)-barcoded antibody. The affinity probes may comprise fluorophore- or (DNA)-barcoded single-chain variable fragments, nanobodies, ligands, aptamers, affibodies, small molecule binders, target binding peptides and proteins, and oligonucleotide probes.

In an embodiment, a pan-protein staining is performed. The pan-protein staining may be performed at any step of the method after the provision of the tissue sample of interest. Thus, the pan staining can be performed before or the epoxide treatment, before or after incubation with and gelation of the first expandable hydrogel solution, before or after the first expansion step, before or after hydrogel stabilization, before or after the incubation with and gelation of the second expandable hydrogel solution, or before or after the second expansion step.

In an embodiment, the hydrogel-embedded tissue sample is neutralized with 0.15-0.5% (w/v) APS and 0.15-0.5% v/v TEMED in water. Alternatively, the hydrogel-embedded tissue sample may be neutralized with 0.15-0.5% (w/v) VA044 or with 0.15-0.5% (w/v) VA050.

In an embodiment, the provided tissue sample is biological sample comprising an organ, a tissue section, an *in vitro* tissue culture, an *in vitro* three-dimensional organ culture, an *in vitro* cell culture or an *in vitro* cultured functional group of cells. In an embodiment, the tissue sample is provided as a tissue section. In an embodiment, the tissue sample is a brain tissue sample. Such tissue section may preferably have a thickness of around 50-300 µm.

In an embodiment, the method may further comprise analyzing the expanded hydrogel-embedded tissue sample with light microscopy. Light microscopy may comprise bright-field, dark field, wide field, confocal, spinning disc confocal, light sheet and/or line scanning microscopy with signal generation via fluorescence, fluorescence lifetime, harmonic generation, wave mixing, phase contrast, absorption, scattering, polarization, vibrational (Raman) contrast or luminescence.

In a further aspect, the present invention relates to a hydrogel for tissue preservation, traceability of cellular structures and experimental robustness, wherein the hydrogel is prepared according to the above mentioned method.

In a further aspect, the present invention relates to a hydrogel for tissue preservation, traceability of cellular structures and experimental robustness, wherein the hydrogel is prepared by an iterative expansion scheme according to the herein disclosed inventive method. Thus the present invention relates to a hydrogel for tissue preservation, traceability of cellular structures and experimental robustness, wherein the hydrogel is prepared by an iterative expansion scheme comprising
a solution for a first expandable hydrogel comprising
   5-15% (w/v) acrylamide
   7-15% (w/v) sodium acrylate
   0.05-0.1% (w/v) N,N'-methylenebisacrylamide
   0.05-0.2% (w/v) of a polymerization initiator
   0.05-0.2% (w/v) of a polymerization accelerator
   0.0005-0.002% (w/v) TEMPO
a solution for a stabilizing hydrogel comprising
   5-15% (w/v) acrylamide
   0.015-0.05% (w/v) N,N'-methylenebisacrylamide
   0.025-0.075% (w/v) polymerization initiator
   0.025-0.075% (w/v) polymerization accelerator, and
a solution for a second expandable hydrogel comprising
   5-15% (w/v) acrylamide
   12.5-22.5% (w/v) sodium acrylate
   0.015-0.05% (w/v) N,N'-methylenebisacrylamide
   0.025-0.075% (w/v) polymerization initiator
   0.025-0.075% (w/v) polymerization accelerator and,
wherein the first expanded hydrogel is generated by gelation and expansion of the solution for a first expanded hydrogel, such hydrogel is subsequently incubated with the solution for the stabilizing hydrogel followed by gelation and wherein the stabilized hydrogel is incubated with the solution for the second expandable hydrogel followed by gelation and expansion, preferably, wherein a tissue sample is embedded in the expandable first hydrogel solution.

In a preferred embodiment, the hydrogel is prepared by an iterative expansion scheme comprising
a solution for a first expandable hydrogel comprising
   5-15% (w/v) acrylamide
   7-15% (w/v) sodium acrylate
   0.05-0.1% (w/v) N,N'-methylenebisacrylamide
   0.05-0.2% (w/v) APS
   0.05-0.2% (w/v) TEMED
   0.0005-0.002% (w/v) TEMPO
a solution for a stabilizing hydrogel comprising
   5-15% (w/v) acrylamide
   0.015-0.05% (w/v) N,N'-methylenebisacrylamide
   0.025-0.075% (w/v) APS
   0.025-0.075% (w/v) TEMED, and
a solution for a second expandable hydrogel comprising
   5-15% (w/v) acrylamide
   12.5-22.5% (w/v) sodium acrylate
   0.015-0.05% (w/v) N,N'-methylenebisacrylamide
   0.025-0.075% (w/v) APS
   0.025-0.075% (w/v) TEMED and,
wherein the first expanded hydrogel is generated by gelation and expansion of the solution for a first expanded hydrogel, wherein the first expanded hydrogel is subsequently incubated with the solution for the stabilizing hydrogel followed by gelation and wherein the stabilized hydrogel is incubated with the solution for the second expandable hydrogel followed by gelation and expansion, optionally, wherein a tissue sample is embedded in the solution for the first expandable hydrogel.

In a further aspect, the present invention relates to the use of the herein disclosed inventive hydrogel in light microscopy. Light microscopy may comprise bright-field, dark field, wide field, confocal, spinning disc confocal, light sheet and/or line scanning microscopy with signal generation via fluorescence, fluorescence lifetime, harmonic generation, wave mixing, phase contrast, absorption, scattering, polarization, vibrational (Raman) contrast or luminescence.

In another aspect, the present invention relates to a kit, wherein the kit comprises at least three vials,
wherein the first vial comprises
   5-15% (w/v) acrylamide
   7-15% (w/v) sodium acrylate
   0.05-0.1% (w/v) N,N'-methylenebisacrylamide
   0.0005-0.002% (w/v) TEMPO
wherein the second vial comprises
   5-15% (w/v) acrylamide
   0.015-0.05% (w/v) N,N'-methylenebisacrylamide
wherein the third vial comprises
   5-15% (w/v) acrylamide
   12.5-22.5% (w/v) sodium acrylate
   0.015-0.05% (w/v) N,N'-methylenebisacrylamide
the kit optionally further comprising a fourth vial comprising
   0.05-0.2% (w/v) of a polymerization initiator
   0.05-0.2% (w/v) of a polymerization accelerator
and a fifth and sixth vial each comprising
   0.025-0.075% (w/v) polymerization initiator
   0.025-0.075% (w/v) polymerization accelerator, and
wherein prior to use of the kit the fourth vial is combined with the first vial, the fifth vial is combined with the second vial and the sixth vial is combined with the third vial.

### DETAILED DESCRIPTION OF THE INVENTION

### Advantages of the invention

The invention provides independent interpenetrating hydrogels.

The herein disclosed method comprises hydrogel embedding and expansion which homogenizes the sample refractive index and adjusts it to that of water, facilitating acquisition of tissue volumes extended both laterally and along the optical (z)-axis, which is notoriously difficult with other optical super-resolution techniques due to aberrations, scattering and photobleaching. The herein disclosed strategy also allows facile incorporation of multi-color readout of specific molecules.

For connectomic reconstruction, 4x or 10x known expanding single-step approaches did not provide a satisfying performance or straightforward modifications to increase expansion factor (exF) resulted in unstable hydrogels. The present disclosure however, provides an iterative (two-step) expansion scheme optimized for high-fidelity tissue preservation, traceability of tissue structures and experimental robustness, yielding both the required resolution and signal-to-noise ratio when paired with light microscopic readout such as spinning-disc confocal readout. Thus, in contrast to established correlative light/electron microscopy workflows for circuit reconstruction, the present invention delivers molecular information at essentially identical resolution as the structural channel.

The iterative expansion scheme presented herein expands biological specimens about 16×, and enables about 18-20 nm-resolution imaging of cells and tissues on conventional microscopes.

Importantly, with the herein disclosed method the first gel and the stabilizing gel do not comprise a cleavable crosslinker such as N,N'-(1,2-dihydroxyethylene) bisacrylamide (DHEBA). Therefore, after incubating the first hydrogel-tissue hybrid with the solution for the second hydrogel and polymerization of this solution to form the second gel and before expansion of the second gel, there is no need to dissolve the previous gels (first hydrogel and stabilization hydrogel) by cleaving their crosslinkers before expansion of the second hydrogel in water. This is because the present invention is not based on crosslinking between individual hydrogels but ensures their independence by chemically quenching any unreacted hydrogel side chains after each polymerization step.

In other words, in known approaches the information of the sample has to be transferred from the 1st hydrogel to the final hydrogel, since the 1st-, and re-embedding hydrogels are cleaved. In the present approach, there is no need for information transfer, as the molecules of interest (proteins) remain anchored to the first gel throughout.

Since according to the herein disclosed methods the hydrogels are not cleaved, the final sample is composed of three hydrogel networks (1st hydrogel network, re-embedding/stabilizing hydrogel network and 2nd hydrogel network): all together these three networks support each other in the overall sample stability and robustness.

In addition, the present method includes neutralizing steps, i.e., quenching steps, after the first expansion step and the polymerization step of the stabilizing gel, respectively. In the field of imaging, the quenching step has not been reported so far. This neutralizing steps produce tough hydrogels. Without the neutralizing step, the final physical appearance of the hydrogel was perturbed. Perturbed physical appearance also perturbs the ultrastructure of the biological sample. Without being bound to the theory, forces generated by each of the three hydrogel networks may negatively influence each other. The way how to ensure these forces are independent of each other and don't contribute to final physical appearance is to strictly separate hydrogel forces from different hydrogels, i.e. these networks shouldn't be connected in any way or even cross-linked. This is achieved by quenching so far unreacted reactive groups before the respective hydrogel is transferred to the subsequent hydrogel solution.

Slicing of the final hydrogel/tissue hybrid to image larger volumes, because in confocal microscope the working distance of the used objective dictates the V one can image.

Using the hydrogels according to the present description, traceability of even thin neuronal structures including axons and dendritic spines can be shown clearly. The present invention thus provides optimized hydrogel compositions for independent, intercalating hydrogels.

Overall, the herein disclosed method provides for a robust and straightforward workflow, providing the resolution, contrast, and experimental throughput required for connectomic tissue reconstruction. The provided hydrogels preserve tissue ultrastructure for tracing the fine structures, such as axons and spines, and are robust and stable.

### Method of producing independent interpenetrating hydrogels

The present method results in independent interpenetrated hydrogels for use in microscopy, preferably light microscopy. The present method is based on an iterative expansion scheme, in which the structures of a sample may be expanded about 20-fold. Iterative means, that the expansion takes place in more than one step. Here, a sample is embedded and incubated in an expandable solution for a hydrogel, which is subsequently polymerized to form a gel. The gel is then expanded in water. This expansion results in kind of a mesh. After this first expansion the hydrogel is stabilized and only subsequently incubated in a second expandable solution for a hydrogel which intercalates in the space newly opened up by the first expansion. After polymerization of the solution the hydrogel is expanded again. Such iterative process may thus expand biological specimens at least two times, i.e. for about 4.8 (1^{st} expansion) × 3.5 (2^{nd} expansion), or for about 16.8-fold, and enables about 15-20, preferably 18-20 nm (250nm/16.8 diffraction limited resolution) resolution imaging of cells and tissues on conventional microscopes. Because the first hydrogel remains intact within the second hydrogel, the resulting hydrogel comprises interpenetrating independent hydrogels.

### Providing a tissue sample of interest

The first step of the herein disclosed method of preparing a tissue sample for microscopic analysis comprising an iterative expansion scheme is the provision of a tissue sample of interest.

The tissue sample may be of any origin. A tissue sample may be a biological sample such as, but not limited to whole organs, tissue sections, *in vitro* tissue cultures, *in vitro* 3-dimensional organ culture, an *in vitro* cell culture or an *in vitro* cultured functional group of cells. The samples may thus be from a variety of organ and non-organ tissues, including but not limited to brain, lung, liver, kidney, spinal cord, heart, intestine, colon etc.

The sample may be derived from any organism, e.g. from vertebrates and invertebrates. An exemplary tissue sample may be provided from any subject comprising an animal, a human, a fish, a reptile. A tissue sample may also be derived from a plant or fungus. In some embodiments, samples are derived from humans, animals such as dogs or cats, agricultural animals such as cows, sheep and pigs, rodents such as rats or mice, zoo animals, primates such as monkeys, and the like. Preferably, the tissue sample is an animal or human tissue sample. A tissue sample may preferably be derived from an organ of an animal or human.

In an embodiment, the tissue sample may be derived from a brain, for instance from an animal brain or a human brain. Exemplary, the tissue sample may be derived from a mouse brain. The skilled person is aware of collecting tissue samples in a way suitable to perform the present method. In an exemplary case, the tissue sample is derived from a mouse brain. In such case, the mouse brain may be harvested from a transcardially perfused mouse and post-fixed using an acrylamide solution. Post-fixation of tissue samples is optional. However the acrylamide concentration in the perfusion step may be optimized for minimal ultrastructure perturbation.

The provided tissue may thus have been fixated in a solution comprising acrylamide, preferably paraformaldehyde (PFA) and acrylamide. In an embodiment, the tissue sample has been fixated in a solution comprising 4 % (weight/volume, (w/v)) PFA and 10% acrylamide (AA) in 1X PBS prior to the provision of the tissue sample for conducting the present method.

The tissue sample may be provided in a size and form suitable for microscopy. Preferably, the tissue sample may be provided in form of a tissue slice, e.g., an organ slice, e.g. prepared by a Vibratome. A tissue sample of interest may be provided in form of a tissue slice with a thickness of between 50 µm and 300 µm. Alternatively, a tissue sample provided in form of a tissue slice may have a thickness of about 10 µm to about 500 µm, preferably of about 25 µm to 350 µm, more preferably from 50 µm to 300 µm. In an embodiment, the tissue sample is a tissue slice with a thickness of about 50 µm. In another embodiment, the tissue sample is a tissue slice with a thickness of about 100 µm. In another embodiment, the tissue sample is a tissue slice with a thickness of about 150 µm. In another embodiment, the tissue sample is a tissue slice with a thickness of about 200 µm. In another embodiment, the tissue sample is a tissue slice with a thickness of about250 µm. In another embodiment, the tissue sample is a tissue slice with a thickness of about 300 µm. In another embodiment, the tissue sample is a tissue slice with a thickness of about 350 µm. In another embodiment, the tissue sample is a tissue slice with a thickness of about 400 µm. The tissue sample may have a thickness of about 500 µm, 1mm, 2mm, 3mm, 4mm, 5mm, 6mm, 7mm, 8mm, 9mm, or 1 cm. In an embodiment, the tissue sample may have a thickness of between 50 µm to 1 mm. In an embodiment, the tissue sample may have a thickness of between 50 µm to 400 µm. In an embodiment, the tissue sample may have a thickness of between 50 µm to 500 µm. In an embodiment, the tissue sample may have a thickness of between 50 µm to 750 µm. In an embodiment, the tissue sample may have a thickness of between 50 µm to 850 µm. In an embodiment, the tissue sample may have a thickness of between 50 µm to 1 mm. In an embodiment, the tissue sample may have a thickness of between 50 µm to 1 cm. Thus, with the herein disclosed methods very thick tissue specimens can be analyzed.

### Treating the tissue sample with an epoxide

According to the herein disclosed method, the provided tissue sample is initially treated with an epoxide. Treatment with an epoxide allows to further functionalize and stabilize cellular molecules. The epoxide is used as a multifunctional anchor. Epoxides suitable for the present method may react with nucleophiles of biological importance, including but not limited to cysteine, histidine, lysine, glutamic acid, tyrosine, and guanine. The epoxide treatment provides polyfunctional reactive groups with a flexible backbone for the formation of intra- and inter molecular bonds, reactivity to various classes of biomolecules, and tightly controllable reaction kinetics under mild conditions. Tissue epoxidation thus cross-links both proteins and nucleic acids while preserving their probe-binding affinities to various antibodies and staining reagents. For instance, GMA is a single-arm epoxide and has an acrydite moiety which may be used as an anchoring agent. Thus GMA attaches acrydite groups which improve anchoring into the hydrogel. TGE having three epoxy rings is a multi-arms epoxide and reacts with multiple partners and thus further fixes and stabilizes cellular proteins during downstream processing. Acrydite groups may subsequently copolymerized with a first expandable acrylamide/sodium acrylate hydrogel, thus anchoring cellular molecules to the hydrogel, as described below.

The tissue sample may be transferred to a solution comprising the epoxide or epoxide mixture, respectively. The tissue sample is incubated in such solution for about 2 h to 5 h, preferably for about 3 h. Preferably, incubation is performed at 37°C. Concentration of the respective epoxide may vary dependent on the tissue sample. Exemplary epoxide concentrations are in a range of between about 0.05 to about 0.15 % (w/v) for tissue samples with a thickness of around 50 µm and in a range of between about 0.75 to about 1.25 % (w/v) for tissue samples with a thickness of around 300 µm. The person skilled in the art will be able to adjust the concentrations to the size of the tissue sample.

The epoxide may be any epoxide known in the art. The epoxide may preferably be selected from the group comprising glycidyl methacrylate (GMA), glycidyl methyl ether (GME), ethylene glycol diglycidyl ether (EGDE), 1,4-butanediol diglycidyl ether (1,4-BDE), dipropylene glycol diglycidyl ether (DGDE), polyglycerol 3-polyglycidyl ether (P3PE), pentaerythritol polyglycidyl ether (PEGE), and/or glycerol triglycidyl ether (TGE) or a mixture of any two or more thereof. The epoxide may be selected from the group consisting of glycidyl methacrylate (GMA), glycidyl methyl ether (GME), ethylene glycol diglycidyl ether (EGDE), 1,4-butanediol diglycidyl ether (1,4-BDE), dipropylene glycol diglycidyl ether (DGDE), polyglycerol 3-polyglycidyl ether (P3PE), pentaerythritol polyglycidyl ether (PEGE), and/or glycerol triglycidyl ether (TGE) or a mixture of any two or more thereof. In an embodiment, the epoxide is glycidyl methacrylate (GMA). The epoxide may be glycidyl methyl ether (GME). The epoxide may be ethylene glycol diglycidyl ether (EGDE). The epoxide may be 1,4-butanediol diglycidyl ether (1,4-BDE). The epoxide may be dipropylene glycol diglycidyl ether (DGDE). The epoxide may be polyglycerol 3-polyglycidyl ether (P3PE). The epoxide may be pentaerythritol polyglycidyl ether (PEGE). The epoxide may be glycerol triglycidyl ether (TGE). The epoxide may be a mixture of any two or more of glycidyl methacrylate (GMA), glycidyl methyl ether (GME), ethylene glycol diglycidyl ether (EGDE), 1,4-butanediol diglycidyl ether (1,4-BDE), dipropylene glycol diglycidyl ether (DGDE), polyglycerol 3-polyglycidyl ether (P3PE), pentaerythritol polyglycidyl ether (PEGE), and/or glycerol triglycidyl ether (TGE).

In a preferable embodiment, the epoxide is glycidyl methacrylate (GMA). In another preferable embodiment, the epoxide is glycerol triglycidyl ether (TGE). In a likewise preferable embodiment, the epoxide used is a mixture of GMA and TGE.

The epoxide may be a polyepoxide. A polyepoxide can crosslink multiple biomolecules to each other, and even multiple parts of a biomolecule to each other, have been shown to help proteins such as fluorescent proteins retain function in tissue specimens under harsh conditions, such as high heating.

An exemplary polyepoxide is trimethylolpropane triglycidyl ether (TMPTE). In an embodiment, GMA and TMPTE may be combined.

In an embodiment, the epoxide may be replaced by or combined with an anchoring compound comprising a N-acryloxysuccinimide (NAS), acrylic acid N-hydroxysuccinimide ester, LabelX, MalphaX and Acryloxyl-X.

### Incubating the tissue sample in a solution for a first expandable hydrogel

The tissue sample is then transferred into a solution for a first expandable hydrogel. The solution for the first expandable hydrogel (first expandable hydrogel solution) comprises acrylamide, sodium acrylate, N,N'-methylenebisacrylamide, a polymerization initiator, a polymerization accelerator and TEMPO. N,N'-methylenebisacrylamide is used as a crosslinker to cross link poly(acrylamide/sodium acrylate) co-polymer.

Acrylamide is present in the solution for a first expandable hydrogel in a concentration of about 5 to about 15 % (w/v). Thus, acrylamide may be present in a concentration of 5 to 15 %(w/v). Preferably, acrylamide may be present in a concentration of between about 7 to about 13 % (w/v). More preferably, acrylamide may be present in a concentration of between about 9.5 to about 10.5 % (w/v). More preferably, acrylamide may be present in a concentration of about 10 % (w/v). Alternatively, acrylamide may be present in the solution at a concentration of 5, 6, 7, 8, 9, 10, 11, 12, 13, 14, or 15 % (w/v).

Sodium acrylate is present in the solution for a first expandable hydrogel in a concentration of about 7 to about 15 %(w/v). Thus, sodium acrylate may be present in a concentration of 7 to 15 %(w/v). Preferably, sodium acrylate may be present in a concentration of between about 10 to about 13 % (w/v). More preferably, sodium acrylate may be present in a concentration of between about 12 to about 13 % (w/v). More preferably, acrylamide may be present in a concentration of about 12.5 % (w/v). Alternatively, sodium acrylate may be present in the solution at a concentration of 7, 8, 9, 10, 11, 12, 13, 14, or 15 % (w/v).

N,N'-methylenebisacrylamide (BIS) is present in the solution for a first expandable hydrogel in a concentration of about 0.05 to about 0.1 %(w/v). Thus, N,N'-methylenebisacrylamide (BIS) may be present in a concentration of 0.05 to 0.1 %(w/v). Preferably, BIS may be present in a concentration of between about 0.06 to about 0.08 % (w/v). More preferably, BIS may be present in a concentration of between about 0.07 to about 0.08 % (w/v). More preferably, BIS may be present in a concentration of about 0.075 % (w/v). Alternatively, BIS may be present in the solution at a concentration of 0.05, 0.055, 0.06, 0.065, 0.07, 0.075, 0.08, 0.085, 0.09, 0.095, or 0.10 % (w/v).

The concentration of the polymerization initiator present in the solution for a first expandable hydrogel depends on what type of polymerization initiator is used. Typically, the polymerization initiator is present in the solution for a first expandable hydrogel in a concentration of about 0.05 to about 0.2 % (w/v). Thus, the polymerization initiator may be present in a concentration of 0.05 to 0.2 % (w/v). The polymerization initiator may be ammonium persulfate (APS). APS may be present in the solution for a first expandable hydrogel in a concentration of about 0.05 to about 0.2 % (w/v). Preferably, APS may be present in a concentration of between about 0.10 to about 0.18 % (w/v). More preferably, APS may be present in a concentration of between about 0.125 to about 0.175 % (w/v). More preferably, APS may be present in a concentration of about 0.15 % (w/v). Alternatively, APS may be present in the solution at a concentration of 0.05, 0.06, 0.07, 0.08, 0.09, 0.095, 0.10, 0.11, 0.12, 0.13, 0.14, 0.15, 0.16, 0.17, 0.18, 0.19, or 2.0% (w/v).

Alternatively, the polymerization initiator is VA044 or VA050. In such case, no additional polymerization accelerator is needed. Therefore, the polymerization initiator and the polymerization accelerator present in the solution for the first expanding hydrogel may be replaced by VA044 or VA050, respectively. VA044 may be present in the solution for a first expandable hydrogel in a concentration of about 0.05 to about 0.2 % (w/v). Preferably, VA044 may be present in a concentration of between about 0.10 to about 0.18 % (w/v). More preferably, VA044 may be present in a concentration of between about 0.125 to about 0.175 % (w/v). More preferably, VA044 may be present in a concentration of about 0.15 % (w/v). Alternatively, VA044 may be present in the solution at a concentration of 0.05, 0.06, 0.07, 0.08, 0.09, 0.095, 0.10, 0.11, 0.12, 0.13, 0.14, 0.15, 0.16, 0.17, 0.18, 0.19, or 2.0 % (w/v).

VA050 may be present in the solution for a first expandable hydrogel in a concentration of about 0.05 to about 0.2 % (w/v). Preferably, VA050 may be present in a concentration of between about 0.10 to about 0.18 % (w/v). More preferably, VA050 may be present in a concentration of between about 0.125 to about 0.175 % (w/v). More preferably, VA050 may be present in a concentration of about 0.15 % (w/v). Alternatively, VA050 may be present in the solution at a concentration of 0.05, 0.06, 0.07, 0.08, 0.09, 0.095, 0.10, 0.11, 0.12, 0.13, 0.14, 0.15, 0.16, 0.17, 0.18, 0.19, or 2.0 % (w/v).

Alternatively, light-triggered polymerization initiators may be used such as 2-oxoglutaric acid. This polymerization initiator may added to the solution in a concentration of 0.01 to 0.05 mol%. Polymerization is initiated by UV-light, preferably 365 nm UV-light. If such polymerization initiator is used, no additional polymerization accelerator is used.

The concentration of the polymerization accelerator present in the solution for a first expandable hydrogel depends on what type of polymerization accelerator is used. Typically, the polymerization accelerator is present in the solution for a first expandable hydrogel in a concentration of about 0.05 to about 0.2 % (w/v). Thus, the polymerization accelerator may be present in a concentration of 0.05 to 0.2 % (w/v). The polymerization accelerator may be tetramethylethylenediamine (TEMED). TEMED may be present in the solution for a first expandable hydrogel in a concentration of about 0.05 to about 0.2 % (w/v). Preferably, TEMED may be present in a concentration of between about 0.10 to about 0.18 % (w/v). More preferably, APS may be present in a concentration of between about 0.125 to about 0.175 % (w/v). More preferably, TEMED may be present in a concentration of about 0.15 % (w/v). Alternatively, TEMED may be present in the solution at a concentration of 0.05, 0.06, 0.07, 0.08, 0.09, 0.095, 0.10, 0.11, 0.12, 0.13, 0.14, 0.15, 0.16, 0.17, 0.18, 0.19, or 2.0% (w/v).

TEMPO ((2,2,6,6-tetramethylpiperidin-1-yl)oxidanyl) is present in the solution for a first expandable hydrogel in a concentration of about 0.0005 to about 0.002 %(w/v). Thus, TEMPO may be present in a concentration of 0.0005 to 0.002 %(w/v). Preferably, TEMPO may be present in a concentration of between about 0.0008 to about 0.0015 % (w/v). More preferably, TEMPO may be present in a concentration of between about 0.0009 to about 0.0012 % (w/v). More preferably, TEMPO may be present in a concentration of about 0.001 % (w/v). Alternatively, TEMPO may be present in the solution at a concentration of 0.0005, 0.0006, 0.0007, 0.0008, 0.0009, 0.00095, 0.001, 0.0011, 0.0012, 0.0013, 0.0014, 0.0015, 0.0016, 0.0017, 0.0018, 0.0019, or 0.002 % (w/v).

The tissue sample is incubated in the solution for a first expandable hydrogel. This means that the tissue sample is placed in the solution in a way that the sample is completely covered at all sides with the solution. Incubation time varies dependent of the size of the tissue sample. For example, for about 50 µm samples incubation time is about 30-45 minutes. For larger samples of, e.g. about 300 µm incubation time may be about 75-90 minutes. Preferably, incubation takes place with gentle agitation.

### Allowing the solution for the first hydrogel to gel

The solution for the first hydrogel incorporating the tissue sample was subsequently allowed to gel, i.e. to polymerize. Gelation may be carried out by increasing the temperature of the solution for the first expandable hydrogel including the tissue sample to 37°C. This may preferably be performed in a humidified chamber. As the tissue sample is present within the solution, the gelation / polymerization of the hydrogel solution results in a "hydrogel-embedded tissue sample" or "tissue-hydrogel hybrid".

### Denaturing or digesting the hydrogel-embedded tissue sample

After gelation, the hydrogel-embedded tissue sample is denatured or digested.

Denaturation may be performed by heat and chemical denaturation to disrupt mechanical cohesiveness while retaining cellular proteins. Thus, for the denaturation step, the hydrogel-embedded tissue sample is transferred in a denaturation buffer. The denaturation buffer may comprise SDS. More preferably, the denaturation buffer comprises SDS, NaCl, and TRIS HCl in H₂O at a pH of 9. Even more preferably, the denaturation buffer comprises 200 mM SDS, 200 mM NaCl, and 50 mM TRIS HCl in ddH₂O, wherein the pH of the buffer is adjusted to 9 with NaOH. The hydrogel-embedded tissue sample is incubated in the denaturation buffer. Preferably, the hydrogel-embedded tissue sample is incubated in the denaturation buffer at 95°C. Incubation time may vary depending on the size of the tissue sample. Incubation may be for about 60 to 120 minutes, preferably for about 100 minutes for samples with a thickness of about 50 µm. Tissue samples of about 300 µm may be incubated for about 4 hours.

Alternatively, the hydrogel-embedded tissue sample may be digested with a protease in order to disrupt mechanical cohesiveness while retaining cellular proteins. Thus, for the digestion step, the hydrogel-embedded tissue sample is transferred in a digestion-buffer comprising a protease. Preferably protease K may be used. More preferably, proteinase K at a concentration of 8 units/mL. The digestion buffer may be applied directly to the hydrogel in about ten times volume excess. Incubation with the digestion-buffer may be for about 6-13 hours, preferably about 12 hours.

### Expanding the hydrogel-embedded tissue sample

The hydrogel-embedded tissue sample is subsequently expanded by placing the hydrogel-embedded tissue sample in water. The water is preferably pure water. The term "pure water" is interchangeable with distilled water, ddH₂O, MilliQ. water, ultrapure water, double deionized water, etc. Preferably the water is free from DNases, RNases and Proteases. During expansion, the water may be regularly replaced with fresh water, e.g. every 20 minutes. Expansion is completed when no further increase of the size of the hydrogel-embedded tissue sample is observable.

Expansion means here a three dimensional broadening of the hydrogel-embedded tissue sample, i.e. broadening in X, Y, and Z directions. Expansion of the first hydrogel may be around 1-fold to around 7-fold, preferably between around 3-fold and 5-fold, more preferably around 4-fold to about 5-fold, or around 4.8-fold compared to the initial size of the tissue sample. An exemplary expansion is a 3-, 3.5-, 4-, 4.5-, 4.8-, 5-, 5.5-, 6-, 6.5-, or 7-fold expansion of the initial size of the hydrogel-embedded tissue sample. In a preferred embodiment, the hydrogel-embedded tissue sample is expanded to around 4.8-fold after the first expansion step.

### Neutralizing the hydrogel-embedded tissue sample - quenching

The neutralization step is needed for the production of truly independent but intercalating hydrogels. In contrast to prior works, the present method is not based on crosslinking between individual hydrogels but ensures their independence by chemically quenching any unreacted hydrogel side chains after each polymerization, i.e., after the gelation and expansion steps.

Neutralization is performed by neutralizing any unreacted double bonds of BIS and other reactive groups remaining after polymerization of the first hydrogel to ensure that it will not react with the following stabilizing and second expandable hydrogels. Neutralization is performed by incubating the first hydrogel in a solution comprising the polymerization initiator and polymerization accelerator. The hydrogel may be neutralized in 0.15-0.5% w/v polymerization initiator and 0.15-0.5% v/v polymerization accelerator in water. Preferably, hydrogel may be neutralized in 0.15-0.5% w/v APS and 0.15-0.5% v/v TEMED in water. Alternatively, hydrogel may be neutralized in 0.15-0.5% w/v VA044 or in 0.15-0.5% w/v VA050.

Concentration of the polymerization initiator and polymerization accelerator may be higher than the respective concentration in the solution for the first expandable hydrogel. Preferably, the concentration of the polymerization initiator and polymerization accelerator is about 25-30% higher than their respective concentrations in the solution for the first expandable hydrogel. Thus, in an embodiment in which 0.15 % (w/v) APS and 0.15 % (w/v) TEMED are comprised in the solution for the first expandable hydrogel, the expanded hydrogel is neutralized in a solution comprising 0.2% (w/v) APS and 0.2% (w/v) TEMED in water. Preferably, the neutralization solution comprises 0.2% (w/v) of the polymerization initiator and 0.2 % (w/v) of the polymerization accelerator in water.

### Incubating the hydrogel-embedded tissue sample in a solution for a stabilizing hydrogel

The hydrogel-embedded tissue sample is then transferred to a solution for a stabilizing hydrogel (stabilizing monomer solution) in order to stabilize this first hydrogel. This solution is not expandable. Such non-expandable stabilizing hydrogel prevents shrinkage during subsequent application of the second expandable hydrogel which intercalates with the first two gels, i.e. the first expanded hydrogel incorporated in the stabilizing hydrogel.

The solution for a stabilizing hydrogel comprises acrylamide, N,N'-methylenebisacrylamide, a polymerization initiator, and a polymerization accelerator. Preferably, the solution comprises 5-15% (w/v) acrylamide, 0.015-0.05% (w/v) N,N'-methylenebisacrylamide, 0.025-0.075% (w/v) polymerization initiator, and 0.025-0.075% (w/v) polymerization accelerator.

In embodiments, the components of the solution for a stabilizing hydrogel may have the following concentrations.

Acrylamide is present in the solution for a stabilizing hydrogel in a concentration of about 5 to about 15 % (w/v). Thus, acrylamide may be present in a concentration of 5 to 15 %(w/v). Preferably, acrylamide may be present in a concentration of between about 8 to about 11 % (w/v). More preferably, acrylamide may be present in a concentration of between about 9.5 to about 10.5 % (w/v). More preferably, acrylamide may be present in a concentration of about 10 % (w/v). Alternatively, acrylamide may be present in the solution at a concentration of 5, 6, 7, 8, 8.5, 9, 9.5, 10, 10.5, 11, 11.5, 12, 13, 14, or 15 % (w/v).

N,N'-methylenebisacrylamide (BIS) is present in the solution for a stabilizing hydrogel in a concentration of about 0.015 to about 0.05 % (w/v). Thus, N,N'-methylenebisacrylamide (BIS) may be present in a concentration of 0.015 to 0.05 % (w/v). Preferably, BIS may be present in a concentration of between about 0.02 to about 0.04 % (w/v). More preferably, BIS may be present in a concentration of between about 0.02 to about 0.03 % (w/v). More preferably, BIS may be present in a concentration of about 0.025 % (w/v). Alternatively, BIS may be present in the solution at a concentration of 0.015, 0.02, 0.025, 0.03, 0.035, 0.04, 0.045, or 0.05 % (w/v).

The concentration of the polymerization initiator present in the solution for a stabilizing hydrogel depends on what type of polymerization initiator is used. Typically, the polymerization initiator is present in the solution for a stabilizing hydrogel in a concentration of about 0.025 to about 0.075 % (w/v). Thus, the polymerization initiator may be present in a concentration of 0.025 to 0.075 % (w/v). The polymerization initiator may be ammonium persulfate (APS). APS may be present in the solution for a stabilizing hydrogel in a concentration of about 0.025 to about 0.075 % (w/v). Preferably, APS may be present in a concentration of between about 0.03 to about 0.06 % (w/v). More preferably, APS may be present in a concentration of between about 0.04 to about 0.06 % (w/v). More preferably, APS may be present in a concentration of about 0.05 % (w/v). Alternatively, APS may be present in the solution at a concentration of 0.025, 0.03, 0.035, 0.04, 0.045, 0.05, 0.055, 0.06, 0.065, 0.07, or 0.075 % (w/v).

Alternatively, the polymerization initiator is VA044 or VA050. In such case, no additional polymerization accelerator is needed. Therefore, the polymerization initiator and the polymerization accelerator present in the solution for a stabilizing hydrogel may be replaced by VA044 or VA050. VA044 may be present in the solution for a stabilizing hydrogel in a concentration of about 0.025 to about 0.075 % (w/v). VA044 may be present in the solution for a stabilizing hydrogel in a concentration of about 0.025 to about 0.075 % (w/v). Preferably, VA044 may be present in a concentration of between about 0.03 to about 0.06 % (w/v). More preferably, VA044 may be present in a concentration of between about 0.04 to about 0.06 % (w/v). More preferably, VA044 may be present in a concentration of about 0.05 % (w/v). Alternatively, VA044 may be present in the solution at a concentration of 0.025, 0.03, 0.035, 0.04, 0.045, 0.05, 0.055, 0.06, 0.065, 0.07, or 0.075 % (w/v).

VA050 may be present in the solution for a stabilizing hydrogel in a concentration of about 0.025 to about 0.075 % (w/v). VA050 may be present in the solution for a stabilizing hydrogel in a concentration of about 0.025 to about 0.075 % (w/v). Preferably, VA050 may be present in a concentration of between about 0.03 to about 0.06 % (w/v). More preferably, VA050 may be present in a concentration of between about 0.04 to about 0.06 % (w/v). More preferably, VA050 may be present in a concentration of about 0.05 % (w/v). Alternatively, VA050 may be present in the solution at a concentration of 0.025, 0.03, 0.035, 0.04, 0.045, 0.05, 0.055, 0.06, 0.065, 0.07, or 0.075 % (w/v).

Alternatively, light-triggered polymerization initiators may be used such as 2-oxoglutaric acid. This polymerization initiator may added to the solution in a concentration of 0.01 to 0.05 mol%. Polymerization is initiated by UV-light, preferably 365 nm UV-light. If such polymerization initiator is used, no additional polymerization accelerator is used.

The concentration of the polymerization accelerator present in the solution for a stabilizing hydrogel depends on what type of polymerization accelerator is used. Typically, the polymerization accelerator is present in the solution for a stabilizing hydrogel in a concentration of about 0.025 to about 0.075 % (w/v). Thus, the polymerization accelerator may be present in a concentration of 0.025 to 0.075 % (w/v). The polymerization accelerator may be tetramethylethylenediamine (TEMED). TEMED may be present in the solution for a stabilizing hydrogel in a concentration of about 0.025 to about 0.075 % (w/v). Preferably, TEMED may be present in a concentration of between about 0.03 to about 0.06 % (w/v). More preferably, TEMED may be present in a concentration of between about 0.04 to about 0.06 % (w/v). More preferably, TEMED may be present in a concentration of about 0.05 % (w/v). Alternatively, TEMED may be present in the solution at a concentration of 0.025, 0.03, 0.035, 0.04, 0.045, 0.05, 0.055, 0.06, 0.065, 0.07, or 0.075 % (w/v).

The hydrogel-embedded tissue sample is incubated in the solution for a stabilizing hydrogel. Incubation is preferably performed at cold temperatures, preferably, on ice water. The tissue sample is placed in the solution in a way that the sample is completely covered at all sides with the solution, preferably incubation is performed with gentle agitation. Incubation time varies dependent on the thickness of the tissue sample from about 3 hours (50 µm) to about 5 hours (300 µm).

### Allowing the solution for a stabilizing hydrogel to gel

The solution for the stabilizing hydrogel incorporating the hydrogel-embedded tissue sample was subsequently allowed to gel, i.e. to polymerize. This has been done at a temperature of about 37°C. Preferably, gelation is carried out in a pre-warmed humidified chamber.

As the hydrogel-embedded tissue sample is present within the solution, the gelation / polymerization of the hydrogel solution results in a stabilized hydrogel-embedded tissue sample. Such non-expandable stabilizing gel prevents shrinkage during subsequent application of the second expandable hydrogel, which intercalates with the first two gels, i.e. the first expandable hydrogel present in the stabilizing gel.

### Neutralizing the hydrogel-embedded tissue sample - quenching

The neutralization step is needed for the production of truly independent but intercalating hydrogels. In contrast to prior works, the present method is not based on crosslinking between individual hydrogels but ensures their independence by chemically quenching any unreacted hydrogel side chains after each polymerization, i.e., after the gelation and expansion steps.

Neutralization is performed by neutralizing remaining unreacted double bonds of BIS or any other reactive groups remaining in the stabilizing hydrogel network to ensure that the hydrogel network will not react with the following second expandable hydrogel network. Neutralization is performed by incubating the hydrogel-embedded tissue sample in a solution comprising the polymerization initiator and polymerization accelerator. The hydrogel may be neutralized in 0.15-0.5% w/v polymerization initiator and 0.15-0.5% v/v polymerization accelerator in water. Preferably, hydrogel may be neutralized in 0.15-0.5% w/v APS and 0.15-0.5% v/v TEMED in water. Alternatively, hydrogel may be neutralized in 0.15-0.5% w/v VA044 or in 0.15-0.5% w/v VA050.

Concentration of the polymerization initiator and polymerization accelerator may be higher than the respective concentrations in the solution for the stabilizing hydrogel. Preferably, the concentration of the polymerization initiator and polymerization accelerator is about four times higher than their respective concentrations in the solution for the stabilizing hydrogel. Thus, in an embodiment in which 0.05 % (w/v) APS and 0.05 % (w/v) TEMED are comprised in the solution for stabilizing hydrogel, the expanded hydrogel is neutralized in a solution comprising 0.2% (w/v) APS and 0.2% (w/v) TEMED in water. Preferably, the neutralization solution comprises 0.2% (w/v) of the polymerization initiator and 0.2 % (w/v) of the polymerization accelerator in water.

### Incubating the hydrogel-embedded tissue sample in a solution for a second expandable hydrogel

The stabilized and neutralized hydrogel-embedded tissue sample is then transferred into a solution for a second expandable hydrogel. The solution for the second expandable hydrogel (second expandable hydrogel solution) comprises acrylamide, sodium acrylate, N,N'-methylenebisacrylamide, a polymerization initiator, and a polymerization accelerator. N,N'-methylenebisacrylamide is used as a crosslinker to cross link poly(acrylamide/sodium acrylate) co-polymer.

Acrylamide is present in the solution for a second expandable hydrogel in a concentration of about 5 to about 15 % (w/v). Thus, acrylamide may be present in a concentration of 5 to 15 %(w/v). Preferably, acrylamide may be present in a concentration of between about 8 to about 11 % (w/v). More preferably, acrylamide may be present in a concentration of between about 9.5 to about 10.5 % (w/v). More preferably, acrylamide may be present in a concentration of about 10 % (w/v). Alternatively, acrylamide may be present in the solution at a concentration of 5, 6, 7, 8, 9, 9.5, 10, 10.5, 11, 12, 13, 14, or 15 % (w/v).

Sodium acrylate is present in the solution for a second expandable hydrogel in a concentration of about 12.5 to about 22.5 %(w/v). Thus, sodium acrylate may be present in a concentration of 12.5 to 22.5 %(w/v). Preferably, sodium acrylate may be present in a concentration of between about 17 to about 20 % (w/v). More preferably, sodium acrylate may be present in a concentration of between about 18.5 to about 19.5 % (w/v). More preferably, acrylamide may be present in a concentration of about 19 % (w/v). Alternatively, sodium acrylate may be present in the solution at a concentration of 12.5, 13, 13.5, 14, 14.5, 15, 15.5, 16, 16.5, 17, 17.5, 18, 18.5, 19, 19.5, 20, 20.5, 21, 21.5, 22, or 22.5 % (w/v).

N,N'-methylenebisacrylamide (BIS) is present in the solution for a second expandable hydrogel in a concentration of about 0.015 to about 0.05 % (w/v). Thus, N,N'-methylenebisacrylamide (BIS) may be present in a concentration of 0.015 to 0.05 % (w/v). Preferably, BIS may be present in a concentration of between about 0.02 to about 0.04 % (w/v). More preferably, BIS may be present in a concentration of between about 0.02 to about 0.03 % (w/v). More preferably, BIS may be present in a concentration of about 0.025 % (w/v). Alternatively, BIS may be present in the solution at a concentration of 0.015, 0.02, 0.025, 0.03, 0.035, 0.04, 0.045, or 0.05 % (w/v).

The concentration of the polymerization initiator present in the solution for a second expandable hydrogel depends on what type of polymerization initiator is used. Typically, the polymerization initiator is present in the solution for a second expandable hydrogel in a concentration of about 0.025 to about 0.075 % (w/v). Thus, the polymerization initiator may be present in a concentration of 0.025 to 0.075 % (w/v). The polymerization initiator may be ammonium persulfate (APS). APS may be present in the solution for a second expandable hydrogel in a concentration of about 0.025 to about 0.075 % (w/v). Preferably, APS may be present in a concentration of between about 0.03 to about 0.06 % (w/v). More preferably, APS may be present in a concentration of between about 0.04 to about 0.06 % (w/v). More preferably, APS may be present in a concentration of about 0.05 % (w/v). Alternatively, APS may be present in the solution at a concentration of 0.025, 0.03, 0.035, 0.04, 0.045, 0.05, 0.055, 0.06, 0.065, 0.07, or 0.075 % (w/v).

Alternatively, the polymerization initiator is VA044 or VA050. In such case, no additional polymerization accelerator is needed. Therefore, the polymerization initiator and the polymerization accelerator present in the solution for a second expandable hydrogel may be replaced by VA044 or VA050. VA044 may be present in the solution for a second expandable hydrogel in a concentration of about 0.025 to about 0.075 % (w/v). VA044 may be present in the solution for a second expandable hydrogel in a concentration of about 0.025 to about 0.075 % (w/v). Preferably, VA044 may be present in a concentration of between about 0.03 to about 0.06 % (w/v). More preferably, VA044 may be present in a concentration of between about 0.04 to about 0.06 % (w/v). More preferably, VA044 may be present in a concentration of about 0.05 % (w/v). Alternatively, VA044 may be present in the solution at a concentration of 0.025, 0.03, 0.035, 0.04, 0.045, 0.05, 0.055, 0.06, 0.065, 0.07, or 0.075 % (w/v).

VA050 may be present in the solution for a second expandable hydrogel in a concentration of about 0.025 to about 0.075 % (w/v). VA050 may be present in the solution for a second expandable hydrogel in a concentration of about 0.025 to about 0.075 % (w/v). Preferably, VA050 may be present in a concentration of between about 0.03 to about 0.06 % (w/v). More preferably, VA050 may be present in a concentration of between about 0.04 to about 0.06 % (w/v). More preferably, VA050 may be present in a concentration of about 0.05 % (w/v). Alternatively, VA050 may be present in the solution at a concentration of 0.025, 0.03, 0.035, 0.04, 0.045, 0.05, 0.055, 0.06, 0.065, 0.07, or 0.075 % (w/v).

Alternatively, light-triggered polymerization initiators may be used such as 2-oxoglutaric acid. This polymerization initiator may added to the solution in a concentration of 0.01 to 0.05 mol%. Polymerization is initiated by UV-light, preferably 365 nm UV-light. If such polymerization initiator is used, no additional polymerization accelerator is used.

The concentration of the polymerization accelerator present in the solution for a second expandable hydrogel depends on what type of polymerization accelerator is used. Typically, the polymerization accelerator is present in the solution for a second expandable hydrogel in a concentration of about 0.025 to about 0.075 % (w/v). Thus, the polymerization accelerator may be present in a concentration of 0.025 to 0.075 % (w/v). The polymerization accelerator may be tetramethylethylenediamine (TEMED). TEMED may be present in the solution for a second expandable hydrogel in a concentration of about 0.025 to about 0.075 % (w/v). Preferably, TEMED may be present in a concentration of between about 0.03 to about 0.06 % (w/v). More preferably, TEMED may be present in a concentration of between about 0.04 to about 0.06 % (w/v). More preferably, TEMED may be present in a concentration of about 0.05 % (w/v). Alternatively, TEMED may be present in the solution at a concentration of 0.025, 0.03, 0.035, 0.04, 0.045, 0.05, 0.055, 0.06, 0.065, 0.07, or 0.075 % (w/v).

The stabilized hydrogel-embedded tissue sample is incubated in the solution for a second expandable hydrogel. This means that the stabilized hydrogel-embedded tissue sample is placed in the solution in a way that the sample is completely covered at all sides with the solution. Preferably, incubation takes place at cold temperatures, preferably on ice water. Incubation time varies dependent on the thickness of the tissue sample. For instance, incubation time may be about 3-3.5 hours (50 µm), or about 5 hours (300 µm).

### Allowing the solution for the second hydrogel to gel

The solution for the second hydrogel comprising the stabilized hydrogel-embedded tissue sample was subsequently allowed to gel, i.e. to polymerize. Gelation may be carried out at 37 °C, preferably in a pre-warmed (37 °C) humidified chamber. Gelation time may vary dependent in the volume of the solution. Gelation time may preferably be about 2 hours.

### Expanding the hydrogel-embedded tissue sample

The hydrogel-embedded tissue sample is subsequently expanded by placing the hydrogel-embedded tissue sample in water. The water is preferably pure water. The term "pure water" is interchangeable with distilled water, ddH₂O, MilliQ water, ultrapure water, double deionized water, etc. Preferably the water is free from DNases, RNases, ions and proteases. During expansion, the water may be regularly replaced with fresh water, e.g. every 20 minutes. Expansion is completed when no further increase of the size of the hydrogel-embedded tissue sample is observable.

Expansion means here a three dimensional broadening of the hydrogel-embedded tissue sample, i.e. broadening in X, Y, and Z directions. Expansion of the second hydrogel may be around 1-fold to around 7-fold, preferably between around 3-fold and 5-fold, more preferably around 4-fold to about 5-fold, compared to the initial size of the hydrogel-embedded tissue sample. An exemplary expansion is a 3-, 3.5-, 4-, 4.5-, 5-, 5.5-, 6-, 6.5-, or 7-fold expansion of the initial size of the hydrogel-embedded tissue sample. In a preferred embodiment, the hydrogel-embedded tissue sample is expanded to around 4.5-fold after the second expansion step. Given that the first expansion of the hydrogel-embedded tissue sample was around 4- to 7-fold, and that the second expansion is again 4- to 7-fold, an overall expansion of at least around 20-fold can be achieved.

Given the above, the present invention provides for a method of preparing a tissue sample for microscopic analysis comprising an iterative expansion scheme comprising providing a tissue sample of interest, treating the tissue sample with an epoxide, incubating the tissue sample in a solution for a first expandable hydrogel and subsequently allowing the solution to gel resulting in a hydrogel-embedded tissue sample, denaturing or digesting the hydrogel-embedded tissue sample, expanding the hydrogel-embedded tissue sample by placing the tissue sample in water, neutralizing the hydrogel-embedded tissue sample, incubating in a solution for a stabilizing hydrogel followed by polymerization of the solution to stabilize the hydrogel-embedded tissue sample, neutralizing the hydrogel-embedded tissue sample, incubating the hydrogel-embedded tissue sample in a solution for a second expandable hydrogel and subsequently allowing the solution to gel, and expanding the hydrogel-embedded tissue sample by placing the hydrogel-embedded tissue sample in pure water,
wherein the solution for the first expandable hydrogel comprises
   5-15% (w/v) acrylamide
   7-15% (w/v) sodium acrylate
   0.05-0.1% (w/v) N,N'-methylenebisacrylamide
   0.05-0.2% (w/v) of a polymerization initiator
   0.05-0.2% (w/v) of a polymerization accelerator
   0.0005-0.002% (w/v) TEMPO
wherein the solution for the stabilizing hydrogel comprises
   5-15% (w/v) acrylamide
   0.015-0.05% (w/v) N,N'-methylenebisacrylamide
   0.025-0.075% (w/v) polymerization initiator
   0.025-0.075% (w/v) polymerization accelerator, and
wherein the solution for the second expandable hydrogel comprises
   5-15% (w/v) acrylamide
   12.5-22.5% (w/v) sodium acrylate
   0.015-0.05% (w/v) N,N'-methylenebisacrylamide
   0.025-0.075% (w/v) polymerization initiator
   0.025-0.075% (w/v) polymerization accelerator and,
optionally, wherein a molecule-specific labeling of one or more targets of interest is performed.

In an embodiment, the solution for the first expandable hydrogel comprises
7-13% (w/v) acrylamide
10-13% (w/v) sodium acrylate
0.06-0.08% (w/v) N,N'-methylenebisacrylamide
0.10-0.18% (w/v) of a polymerization initiator
0.10-0.18% (w/v) of a polymerization accelerator
0.0008-0.0015% (w/v) TEMPO,

the solution for the stabilizing hydrogel comprises
   8-11% (w/v) acrylamide
   0.02-0.04% (w/v) N,N'-methylenebisacrylamide
   0.03-0.06% (w/v) polymerization initiator
   0.03-0.06% (w/v) polymerization accelerator, and
the solution for the second expandable hydrogel comprises
   8-11% (w/v) acrylamide
   17-20% (w/v) sodium acrylate
   0.02-0.03% (w/v) N,N'-methylenebisacrylamide
   0.03-0.06% (w/v) polymerization initiator
   0.03-0.06% (w/v) polymerization accelerator.

In another embodiment, the solution for the first expandable hydrogel comprises
9.5-10.5% (w/v) acrylamide
12-13% (w/v) sodium acrylate
0.07-0.08% (w/v) N,N'-methylenebisacrylamide
0.125-0.175% (w/v) of a polymerization initiator
0.125-0.175% (w/v) of a polymerization accelerator
0.0009-0.0012% (w/v) TEMPO,

the solution for the stabilizing hydrogel comprises
   9.5-10.5% (w/v) acrylamide
   0.02-0.03% (w/v) N,N'-methylenebisacrylamide
   0.04-0.06% (w/v) polymerization initiator
   0.04-0.06% (w/v) polymerization accelerator, and
the solution for the second expandable hydrogel comprises
   9.5-10.5% (w/v) acrylamide
   18.5-19.5% (w/v) sodium acrylate
   0.02-0.03% (w/v) N,N'-methylenebisacrylamide
   0.04-0.06% (w/v) polymerization initiator
   0.04-0.06% (w/v) polymerization accelerator.

In a preferred embodiment, the solution for the first expandable hydrogel comprises
10% (w/v) acrylamide
12,5% (w/v) sodium acrylate
0.075% (w/v) N,N'-methylenebisacrylamide
0.15% (w/v) of a polymerization initiator
0.15% (w/v) of a polymerization accelerator
0.001% (w/v) TEMPO,

the solution for the stabilizing hydrogel comprises
   10% (w/v) acrylamide
   0.025% (w/v) N,N'-methylenebisacrylamide
   0.05% (w/v) polymerization initiator
   0.05% (w/v) polymerization accelerator, and
the solution for the second expandable hydrogel comprises
   10% (w/v) acrylamide
   19% (w/v) sodium acrylate
   0.025% (w/v) N,N'-methylenebisacrylamide
   0.05% (w/v) polymerization initiator
   0.05% (w/v) polymerization accelerator.

In another embodiment, the solution for the first expandable hydrogel comprises
5-15% (w/v) acrylamide
7-15% (w/v) sodium acrylate
0.05-0.1% (w/v) N,N'-methylenebisacrylamide
0.05-0.2% (w/v) APS
0.05-0.2% (w/v) TEMED
0.0005-0.002% (w/v) TEMPO

the solution for the stabilizing hydrogel comprises
   5-15% (w/v) acrylamide
   0.015-0.05% (w/v) N,N'-methylenebisacrylamide
   0.025-0.075% (w/vAPS
   0.025-0.075% (w/v) TEMED, and
the solution for the second expandable hydrogel comprises
   5-15% (w/v) acrylamide
   12.5-22.5% (w/v) sodium acrylate
   0.015-0.05% (w/v) N,N'-methylenebisacrylamide
   0.025-0.075% (w/v) APS
   0.025-0.075% (w/v) TEMED.

In an embodiment, the solution for the first expandable hydrogel comprises
7-13% (w/v) acrylamide
10-13% (w/v) sodium acrylate
0.06-0.08% (w/v) N,N'-methylenebisacrylamide
0.10-0.18% (w/v) APS
0.10-0.18% (w/v) TEMED
0.0008-0.0015% (w/v) TEMPO,

the solution for the stabilizing hydrogel comprises
   8-11% (w/v) acrylamide
   0.02-0.04% (w/v) N,N'-methylenebisacrylamide
   0.03-0.06% (w/v) APS
   0.03-0.06% (w/v) TEMED, and
the solution for the second expandable hydrogel comprises
   8-11% (w/v) acrylamide
   17-20% (w/v) sodium acrylate
   0.02-0.03% (w/v) N,N'-methylenebisacrylamide
   0.03-0.06% (w/v) APS
   0.03-0.06% (w/v) TEMED.

In another embodiment, the solution for the first expandable hydrogel comprises
9.5-10.5% (w/v) acrylamide
12-13% (w/v) sodium acrylate
0.07-0.08% (w/v) N,N'-methylenebisacrylamide
0.125-0.175% (w/v) APS
0.125-0.175% (w/v) TEMED
0.0009-0.0012% (w/v) TEMPO,

the solution for the stabilizing hydrogel comprises
   9.5-10.5% (w/v) acrylamide
   0.02-0.03% (w/v) N,N'-methylenebisacrylamide
   0.04-0.06% (w/v) APS
   0.04-0.06% (w/v) TEMED, and
the solution for the second expandable hydrogel comprises
   9.5-10.5% (w/v) acrylamide
   18.5-19.5% (w/v) sodium acrylate
   0.02-0.03% (w/v) N,N'-methylenebisacrylamide
   0.04-0.06% (w/v) APS
   0.04-0.06% (w/v) TEMED.

In the most preferred embodiment, the solution for the first expandable hydrogel comprises
10% (w/v) acrylamide
12,5% (w/v) sodium acrylate
0.075% (w/v) N,N'-methylenebisacrylamide
0.15% (w/v) APS
0.15% (w/v) TEMED
0.001% (w/v) TEMPO,

the solution for the stabilizing hydrogel comprises
   10% (w/v) acrylamide
   0.025% (w/v) N,N'-methylenebisacrylamide
   0.05% (w/v) APS
   0.05% (w/v) TEMED, and
the solution for the second expandable hydrogel comprises
   10% (w/v) acrylamide
   19% (w/v) sodium acrylate
   0.025% (w/v) N,N'-methylenebisacrylamide
   0.05% (w/v) APS
   0.05% (w/v) TEMED.

### Optional molecule-specific labeling of one or more targets of interest

Once the sample is preserved according to the methods provided herein, it may be analyzed for the presence of one or more targets such as proteins and other biomolecules. In order to analyze target structures in the tissue, such structures have to be labeled or marked. Any suitable labeling can be used. For the labeling, target binding partners may be used. The binding partners may be any molecule or compound capable of binding, preferably specifically, to the target of interest. Molecule-specific labeling may be performed at any time point of the hydrogel expansion protocol. For instance, molecule-specific labeling may be performed after the epoxide treatment, after expanding the first hydrogel, or after expanding the second hydrogel.

Pre-expansion immune-labeling, i.e. labeling after the epoxide treatment step, may be performed for distortion analysis measurements. In such case, labeling is carried out after the epoxide treatment step but before incubating the sample in the solution for the first expandable hydrogel. Pre-expansion images may then be acquired on a spinning disc confocal microscope after the first hydrogel gelation step.

A pre-expansion label may be tri-functional, comprising, e.g. a methacryloyl group capable of participating in free radical polymerization, a chemical fluorophore for visualization, and an oligonucleotide that can hybridize to a complementary sequence attached to an affinity tag (such as a secondary antibody) (see, Chen et al., Science, vol. 347(6221), 2015). In an example, the target of interest may first be labeled with a primary antibody and then with a secondary antibody bearing an oligonucleotide. Subsequently, a second oligonucleotide bearing a gel-anchoring moiety (e.g., a 5' acrydite group) and a fluorophore may be applied and anchored to an expandable hydrogel synthesized evenly throughout the specimen. After mechanical homogenization by denaturation with SDS or digestion with protease treatment, the polymer-specimen composite can subsequently be expanded in water.

Preferably, immuno-labeling is performed post-expansion, i.e., either after the expansion of the first expandable hydrogel or after the expansion of the second expandable hydrogel. Immuno-labeling can also be performed after polymerizing the stabilizing gel. Labeling after the first expansion can be performed with a fluorescent label that can be incorporated directly into the polymer network and thus survives the digestion/denaturation of endogenous biomolecules, which is performed in a subsequent step in the herein disclosed method. Preferably, molecule-specific labeling is performed after the expansion of the second tissue-embedded hydrogel, i.e., after completion of the expansion procedure. Such approach allows to take advantage of the molecular decrowding for improved epitope accessibility and leads to a negligible label size relative to the expanded structure sizes.

Molecule-specific labeling comprises immuno-labeling and labeling with other affinity probes. Immuno-labeling may be carried out by using antibodies and/or antigen-binding antibody fragments. The antibodies and fragments thereof may be monoclonal antibodies. Molecule-specific labeling may thus comprise incubation with a primary antibody and a fluorophore-conjugated secondary antibody or a (DNA)-barcoded antibody. Additionally or alternatively, labeling may be carried out by use of affinity probes comprising fluorophore- or (DNA)-barcoded single-chain variable fragments, nanobodies, ligands, peptide aptamers, nucleic acid aptamers, affibodies, small molecule binders, target binding peptides and proteins, and oligonucleotide probes. Immuno-labeling and labeling with affinity probes is well known to the skilled person who is also aware of the respective staining protocols.

As used herein, "antibody" includes full-length antibodies and any antigen binding fragment (e.g., "antigen-binding portion") or single chain thereof. The term "antibody" includes, without limitation, a glycoprotein comprising at least two heavy (H) chains and two light (L) chains inter-connected by disulfide bonds, or an antigen binding portion thereof. Antibodies may be polyclonal or monoclonal; xenogeneic, allogeneic, or syngeneic; or modified forms thereof (e.g., humanized, chimeric).

As used herein, "antigen-binding portion" of an antibody, refers to one or more fragments of an antibody that retain the ability to specifically bind to an antigen. The antigen- binding function of an antibody can be performed by fragments of a full-length antibody. Examples of binding fragments encompassed within the term "antigen-binding portion" of an antibody include (i) a Fab fragment, a monovalent fragment consisting of the VH, VL, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VH and VL domains of a single arm of an antibody, (v) a dAb fragment, which consists of a VH domain; and (vi) an isolated complementarity determining region (CDR) or (vii) a combination of two or more isolated CDRs, which may optionally be joined by a synthetic linker.

As used herein, "peptide aptamer" refers to a molecule with a variable peptide sequence inserted into a constant scaffold protein (see, e.g., Baines et al. Drug Discov. Today 11 :334-341, 2006).

As used herein, "nucleic acid aptamer" refers to a small RNA or DNA molecules that can form secondary and tertiary structures capable of specifically binding proteins or other cellular targets (see, e.g., Ni et al. Curr Med Chem. 18(27): 4206-4214, 2011).

In an embodiment, antibodies and affinity probes may themselves be labeled with detectable labels.

Detectable labels may be fluorophores. Examples of fluorophores include, without limitation, xanthene derivatives (e.g., fluorescein, rhodamine, Oregon green, eosin and Texas red), cyanine derivatives (e.g., cyanine, indocarbocyanine, oxacarbocyanine, thiacarbocyanine and merocyanine), naphthalene derivatives (e.g., dansyl and prodan derivatives), coumarin derivatives, oxadiazole derivatives (e.g., pyridyloxazole, nitrobenzoxadiazole and benzoxadiazole), pyrene derivatives (e.g., cascade blue), oxazine derivatives (e.g., Nile red, Nile blue, cresyl violet and oxazine 170), acridine derivatives (e.g., proflavin, acridine orange and acridine yellow), arylmethine derivatives (e.g., auramine, crystal violet and malachite green), rhodamines and tetrapyrrole derivatives (e.g., porphin, phthalocyanine and bilirubin). Other detectable labels may be used in accordance with the present disclosure, such as, for example, gold nanoparticles or other detectable particles or moieties.

When two or more labeling moieties are used simultaneously, it may be preferable to label such labeling moieties with labels that are spectrally distinct (i.e., that can be distinguished from other label being used simultaneously). In other words, in some embodiments, the labels can be distinguished from each other.

A "target of interest" may be any biomolecule comprising a protein, peptide, epitope, or a nucleic acid such as RNA and DNA. The target may be a cellular or extracellular target. Virtually any target of interest may be imaged using the method disclosed herein provided a suitable labeling moiety exists. The targets may be found in various locations including without limitation at cell the membrane (e.g., membrane bound), in the cytoplasm, in various organelles such as but not limited to the nucleus, and/or at synapses, and the like.

As an example, a target of interest in the tissue sample is labeled with a primary anti-target antibody, washed, and subsequently stained with secondary analyzable antibody.

### Pan-protein staining

The term "pan" is Greek for "whole". That means, a pan protein staining is the labeling of the whole proteome, or proteins in bulk. Thus with a pan staining or also called protein density staining, proteins can be localized to sub-compartments revealed in the contextual pan-stained channel. Thus, with the pan staining, cellular structures can be comprehensively visualized by e.g. mapping the density of (primary) amines present in large quantity on proteins through various amino acid side chains with fluorescent dyes.

Before performing such pan protein staining, the tissue sample may, before the epoxide treatment step, be incubated in a solution of acrylamide and formaldehyde to simultaneously prevent inter-protein crosslinks and tether proteins to the hydrogel. Therefore, in an embodiment, the method includes incubation of the provided tissue sample in a solution comprising acrylamide and formaldehyde.

In an embodiment, pan-protein staining is performed after the epoxide treatment step and before the incubation of the sample in a solution for the first expandable hydrogel, after expansion and neutralization of the first hydrogel the first gel and before the incubation of the hydrogel in the stabilization solution, or after expansion of the second hydrogel. Preferably, pan-protein staining is performed after the expansion of the second hydrogel.

For the pan staining, different labels can be used. In general, pan staining can be performed with fluorescence labelling as well as with other chemical staining comprising NHS ester, maleimide and palimate pan stainings. Pan staining can thus be performed by using N-Hydroxysuccinimide (NHS) ester-dye conjugates, taking advantage of the abundance of primary amines on proteins. An exemplary ester conjugate is NHS ester-Alexa488, NHS ester-ATTO488, NHS ester-ATTO594, NHS ester-ATTO532 or NHS ester-DY634. Alternatively, pan staining can be performed by using azide-functionalized palmitate. Alternatively, pan staining can be performed by using maleimide-ATTO594. Pan staining can exemplary also or additionally be performed using live-cell small molecule dye such as MitoTracker Orange, which covalently binds to proteins in the matrix of mitochondria. DNA-intercalating dyes such as SYTOX Green are equally compatible for the pan staining for labeling the nucleus and mitochondrial nucleoids.

Alternatively or additionally, pan-protein staining may be performed using tetrafluorophenol (TFP), pentafluorophenol (PFP), epoxide reactions, tyramide chemistry, or hydrazide reactions for attaching dyes for fluorescent readout.

### Microscopic readout

The herein disclosed method may further comprise a step of analyzing the expanded hydrogel-embedded tissue sample with light microscopy. Light microscopy comprises bright-field, dark field, wide field, confocal, spinning disc confocal, light sheet and/or line scanning microscopy. The signal generation of the light microscopy may thus comprise signal generation via fluorescence, fluorescence lifetime, harmonic generation, wave mixing, phase contrast, absorption, scattering, polarization, vibrational (Raman) contrast or luminescence.

The sort of light microscopy used for the microscopic analysis of the hydrogel may be chosen depending on the way of labeling of the target(s) of interest present in the tissue sample. As an example, if immuno-labeling was carried out with fluorescent antibodies, the light microscopy may be confocal microscopy with a signal generation via fluorescence.

### The hydrogels

The tissue-embedded hydrogel prepared according to the method disclosed herein features an around 16-fold overall expansion and were mechanically robust, facilitating handling and extended imaging.

The present invention thus provides a hydrogel for tissue preservation, traceability of cellular structures and experimental robustness, wherein the hydrogel is prepared by an iterative expansion scheme comprising
a solution for a first expandable hydrogel comprising
   5-15% w/v acrylamide
   7-15% w/v sodium acrylate
   0.05-0.1% w/v N,N'-methylenebisacrylamide
   0.05-0.2% w/v of a polymerization initiator
   0.05-0.2% w/v of a polymerization accelerator
   0.0005-0.002% w/v TEMPO
a solution for a stabilizing hydrogel comprising
   5-15% w/v acrylamide
   0.015-0.05% w/v N,N'-methylenebisacrylamide
   0.025-0.075% w/v polymerization initiator
   0.025-0.075% w/v polymerization accelerator, and
a solution for a second expandable hydrogel comprising
   5-15% w/v acrylamide
   12.5-22.5% w/v sodium acrylate
   0.015-0.05% w/v N,N'-methylenebisacrylamide
   0.025-0.075% w/v polymerization initiator
   0.025-0.075% w/v polymerization accelerator and,
wherein the first expanded hydrogel is generated by gelation and expansion of the solution for a first expanded hydrogel, such hydrogel is subsequently incubated with the solution for the stabilizing hydrogel followed by gelation and
wherein the stabilized hydrogel is incubated with the solution for the second expandable hydrogel followed by gelation and expansion,
optionally, wherein a tissue sample is embedded in the expandable first hydrogel solution.

Alternatively, the present invention provides a hydrogel for tissue preservation, traceability of cellular structures and experimental robustness, wherein the hydrogel is prepared by an iterative expansion scheme according to the herein disclosed method. All embodiments disclosed for the method are equally referring to the hydrogel of the invention.

### Use of the hydrogel in light microscopy

The herein disclosed hydrogel may be used in light microscopy, i.e. for analyses conducted with a light microscope. Light microscopy comprises bright-field, dark field, wide field, confocal, spinning disc confocal, light sheet and/or line scanning microscopy. The signal generation of the light microscopy may thus comprise signal generation via fluorescence, fluorescence lifetime, harmonic generation, wave mixing, phase contrast, absorption, scattering, polarization, vibrational (Raman) contrast or luminescence.

The sort of light microscope used for the microscopic analysis of the hydrogel may be chosen depending on the way of labeling of the target(s) of interest present in the tissue sample embedded in the hydrogel. As an example, if immuno-labeling was carried out with fluorescent antibodies, the light microscope may be confocal microscope with a signal generation via fluorescence.

As will be apparent to those of skill in the art upon reading this disclosure, each of the individual embodiments described and illustrated herein has discrete components and features which can be readily separated from or combined with the features of any of the other several embodiments without departing from the scope or spirit of the present teachings. Any recited method can be carried out in the order of events recited or in any other order which is logically possible.

### EXAMPLES

### Example 1. Preparing a tissue sample for microscopic analysis

### Transcardial fixative perfusion

Solutions were prepared on the same day and kept at room temperature (RT). Animals were first anaesthetized with isoflurane (1-2 % (volume/volume, v/v)) and then with Ketamine (80-100 mg kg-1 of body weight) and Xylazine (10 mg kg-1) intraperitoneally, combined with Metamizol (200 mg kg-1) subcutaneously for analgesia. After checking for deep anesthesia by toe pinch, they were transcardially perfused at a flow rate of 7 ml min-1 using a peristaltic pump, first with RT 1X PBS for 2 min and then with a solution (RT) containing 4 % (weight/volume, (w/v)) PFA (pH 7.4) and 10% acrylamide (AA) in 1X PBS.

Brains were harvested and post-fixed for ≤12 h at 4 °C with gentle agitation, using the same acrylamide solution.

### Brain tissue processing

Brains were washed 3x in cold (4°C) 1X PBS for ~1 min each with gentle agitation. They were sectioned coronally at 50 µm thickness using a Vibratome (Leica VT 1200 S). Alternatively, sections were prepared at 300 µm thickness. Sections were placed in ice-cold 1X PBS supplemented with 100 mM glycine to quench PFA reactive groups for 6-8 h at 4°C and washed 3x for ~1 min in 4°C 1X PBS. They were stored at 4°C in 1X PBS supplemented with 0.015% NaN₃ for typically up to 3 months.

### Epoxide treatment

Brain sections were washed 2x for 15 min each in 1X PBS at RT with gentle agitation. They were then washed 2x for 15 min each in 100 mM sodium bicarbonate in ddH₂O, pH 8.0, at RT followed by incubation with 0.1% ((w/v)) TGE and 0.1% ((w/v)) GMA in 100 mM sodium bicarbonate in ddH2O (pH 8.0) for 3 h at 37 °C with gentle agitation in a chemical hood. For 300-µm thick slices, TGE and GMA concentration was increased to 1% ((w/v)). Samples were then washed with 1X PBS for 1 h with gentle agitation at RT.

### Hydrogel embedding and expansion

**Table 1: Composition of hydrogels.**

| | Acrylamide (% (w/v)) | Sodium acrylate (% (w/v)) | N, N'-Methylenebisacrylamide (%(w/v)) | APS (% (w/v)) | TEMED (% (w/v)) | TEMPO |
|---|---|---|---|---|---|---|
| 1^{st} expandable hydrogel | 10 | 12.5 | 0.075 | 0.15 | 0.15 | 0.001 |
| Stabilizing hydrogel | 10 | ---- | 0.025 | 0.05 | 0.05 | ---- |
| 2^{nd} expandable hydrogel | 10 | 19 | 0.025 | 0.05 | 0.05 | ---- |

For preparation of the 1^{st} hydrogel solution, AA and sodium acrylate (SA) were mixed in ddH₂O, vortexed and centrifuged at 4500 G for 5 min. Supernatant was transferred to a fresh 50 ml tube and supplemented with N,N'-Methylenebisacrylamide (BIS) stock solution (prepared in ddH₂O) to a final BIS concentration of 0.075% (w/v). After adjustment to the final volume solutions were aliquoted in 2 ml tubes and stored at -20 °C, typically for up to 1 month. Solutions for stabilizing and 2^{nd} expandable hydrogels were prepared similarly, usually freshly before experiments.

### Polymerization of the first expandable hydrogel

In an ice-water bath, the 1^{st} hydrogel monomer solution (see Table 1) was first supplemented with 0.001% TEMPO and then with 0.15% APS and 0.15% TEMED and vortexed. The following sample handling steps were performed in an ice-water bath. Brain sections were pre-incubated with the hydrogel monomer solution for 30-45 min (75-90 min for 300 µm thick slices) with gentle agitation in a 24 well plate. A gelation chamber was assembled from two cover glasses on bottom and top and #1 cover glass strips on either side as spacers (for 300 µm sections: one #1 and one #1.5 cover glass strip). Gelation was then carried out at 37 °C in a humidified chamber for 2h. For optionally acquiring pre-expansion images, the sample was imaged on a spinning disc confocal microscope (Oxford Instruments, Andor Dragonfly) after the first hour of gelation for ~15 min and then further incubated at 37 °C for another ~45 minutes.

### Disruption of mechanical cohesiveness, expansion, hydrogel neutralization

After gelation, the top coverslip was removed and the hydrogel-tissue hybrid (hadrogel-embedded tissue sample) trimmed to the region of interest. Together with the bottom coverslip, it was then transferred to a 5 ml beaker with 2 ml of denaturation buffer (200 mM SDS, 200 mM NaCl, 50 mM TRIS-HCl in ddH2O and pH adjusted to 9.0 with NaOH). The beaker was placed in a larger vessel containing pre-heated water and placed in a water bath at 95 °C for 100 min. For 300 µm thick samples, denaturation time was extended to 4 h, starting at 85 °C and ramped up to 95 °C within ~15 min.

For expansion, samples were placed in ddH₂O, with water being changed every ~20 min until no further increase in gel size was observed.

Unreacted double bonds of the divinyl-cross linker (BIS) remaining after polymerization of the 1^{st} hydrogel were neutralized to ensure that it would not react with the following stabilizing and 2^{nd} expandable hydrogels. For this, the samples were incubated with 0.2% APS plus 0.2% TEMED in ddH2O for 2.5 h at 37°C with gentle agitation, followed by one washing step with agitation in ddH2O for 30 min at 37°C and a similar washing step at RT.

### Immunolabeling

Expanded hydrogels were again trimmed to the region of interest and incubated in 1X PBS for 10 min at RT, shrinking the gel in a 12-well plate. It was then incubated with primary antibodies in 1X PBS at 4°C ON with gentle agitation in a 24-well plate, followed by ≥3 washes for 1 h each in 1X PBS with gentle agitation at RT. Secondary antibody incubation was also performed in 1X PBS at 4°C ON with gentle agitation, followed by ≥3 washes for 1 h each in 1X PBS with gentle agitation at RT. Post-fixation was conducted for 15 min at RT with 4% PFA in 1X PBS, followed by quenching with 100 mM glycine in 1X PBS for 10 min at RT with gentle agitation and subsequent 3x washing in 1X PBS for ~3 min each. Hydrogels were re-expanded in ddH2O in a 12-well plate before further processing. Labeling was optionally evaluated by imaging.

### Polymerization of stabilizing hydrogel and neutralization

Expanded hydrogels were pre-incubated on ice-water for 3-3.5 h or 5 h for 50 µm and 300 µm thick slices, respectively, with the monomer solution of the stabilizing hydrogel (10% ((w/v)) AA, 0.025% ((w/v)) BIS, 0.05% (v/v) TEMED and 0.05% ((w/v)) APS in ddH2O) with gentle agitation in a 12-well plate. After removing excess monomer solution, the hydrogel was sandwiched between a 22 × 22 mm² coverslip placed on a microscopy slide and a 18 mm round coverslip on top of the hydrogel without spacers, and surrounded by monomer solution. Gelation was then carried out for 2 h at 37°C in a pre-warmed (37°C) humidified chamber. Gels were washed with ddH2O for ~30-60 min at RT.

To neutralize remaining unreacted double bonds of the BIS crosslinker in the stabilizing hydrogel network and ensure that the hydrogel network did not react with the following 2^{nd} expandable hydrogel network, the hydrogel-tissue hybrid was incubated with 0.2% APS and 0.2% TEMED in ddH2O for 2.5 h at 37 °C with gentle agitation, followed by washing with agitation in ddH2O for 30 min at 37 °C and a second washing step at RT.

### Polymerization and expansion of second expandable hydrogel

Stabilized and neutralized hydrogels were pre-incubated on ice-water with the monomer solution for the second expandable hydrogel (19% SA, 10% AA, 0.025% ((w/v)) BIS, 0.05% TEMED, 0.05% APS in ddH₂O) in a 12-well plate for 3-3.5 h (50 µm slices), or 5 h (300 µm slices). After removing excess monomer solution, the hydrogel was again sandwiched between a 22 × 22 mm2 coverslip placed on a microscopy slide and a 18 × 18 mm2 coverslip on top of the hydrogel without spacers, and surrounded by monomer solution. Gelation was then carried out for 2 h at 37 °C in a pre-warmed (37 °C) humidified chamber. Hydrogels were washed with 1X PBS for ~30 min at RT.

For expansion, samples were placed in ddH₂O, with water being changed every ~20 min until no further increase in gel size was observed.

### Pan-protein labeling

Pan-protein staining was performed with either 40 µM ATTO 488 NHS-ester or Alexa Fluor 488 NHS-ester in 1X PBS ON at 4 °C with gentle agitation. Hydrogels were optionally washed 3x for 1 h total with 1X PBS, and expanded for up to 4 h before imaging in ddH2O with fluid exchange approximately every hour.

### Example 2. Microscopic analysis of the hydrogel produced in Example 1

A sample in form of a tissue slice has been prepared according to Example 1. The tissue slice was 50 µm thick. A 1.04 Million cubic micrometer (396 × 109 × 22 µm³) tissue volume spanning layers II/III - IV in primary somatosensory cortex (SSP-TR-L2/3-4, Allen brain atlas) was analyzed. Imaging of ~3.5*10⁹ cubic microns physical volume after expansion was accomplished within 6.5 h on a spinning-disc confocal microscope by imaging 132 partially overlapping sub-volumes (arranged in a 6x22 grid) and adapting an automated image fusion algorithm. Individual cells were clearly delineated from each other in the densely packed neuropil and showed rich subcellular structure, including e.g. mitochondria with cristae and the Golgi apparatus. A prominent ring-like periodic pattern at the circumference of a subset of neurites was also observable. The periodicity of 89± 12 nm was highly suggestive of the actin/β-spectrin lattice previously reported with 180 nm periodicity when only one component was selectively labeled, and further corroborated the quantitative nature of the expansion procedure. Zooming in on dendrites and their dendritic spines, specialized postsynaptic structures typical of excitatory synapses, putative synaptic transmission sites highlighted by protein-rich, high-intensity features could be found. Together with the structural appearance of the involved cellular elements, these were highly suggestive of pre- and postsynaptic transmission sites, similar to well-known features at excitatory synapses in chemically fixed EM specimens. Putative pre-synaptic sites displayed protein-dense nanoscale features arranged in a regular pattern spaced 93 ± 15 nm, whereas putative post-synapses displayed more homogeneous "densities", spaced 139 ± 19 nm from the pre-synaptic features.

This indicates that the herein presented technology is capable of comprehensively revealing the cellular constituents of brain tissue across length scales required for dense connectomic reconstruction.

## Claims

1. Method of preparing a tissue sample for microscopic analysis comprising an iterative expansion scheme comprising
providing a tissue sample of interest,
treating the tissue sample with an epoxide,
incubating the tissue sample in a solution for a first expandable hydrogel and subsequently allowing the solution to gel resulting in a hydrogel-embedded tissue sample,
denaturing or digesting the hydrogel-embedded tissue sample,
expanding the hydrogel-embedded tissue sample by placing the hydrogel embedded tissue sample in water,
neutralizing the hydrogel-embedded tissue sample,
incubating the hydrogel-embedded tissue sample in a solution for a stabilizing hydrogel followed by polymerization of the solution for a stabilizing hydrogel to stabilize the hydrogel-embedded tissue sample,
neutralizing the hydrogel-embedded tissue sample,
incubating the hydrogel-embedded tissue sample in a solution for a second expandable hydrogel and subsequently allowing the solution to gel, and
expanding the hydrogel-embedded tissue sample by placing the hydrogel-embedded tissue sample in water,
wherein the solution for the first expandable hydrogel comprises
5-15% (w/v) acrylamide
7-15% (w/v) sodium acrylate
0.05-0.1% (w/v) N,N'-methylenebisacrylamide
0.05-0.2% (w/v) of a polymerization initiator
0.05-0.2% (w/v) of a polymerization accelerator
0.0005-0.002% (w/v) TEMPO
wherein the solution for the stabilizing hydrogel comprises
5-15% (w/v) acrylamide
0.015-0.05% (w/v) N,N'-methylenebisacrylamide
0.025-0.075% (w/v) polymerization initiator
0.025-0.075% (w/v) polymerization accelerator, and
wherein the solution for the second expandable hydrogel comprises
5-15% (w/v) acrylamide
12.5-22.5% (w/v) sodium acrylate
0.015-0.05% (w/v) N,N'-methylenebisacrylamide
0.025-0.075% (w/v) polymerization initiator
0.025-0.075% (w/v) polymerization accelerator and,
optionally, wherein a molecule-specific labeling of one or more targets of interest is performed.

2. The method of claim 1, wherein the epoxide is glycidyl methacrylate (GMA), glycerol triglycidyl ether (TGE), or a mixture thereof, preferably, wherein the epoxide is a mixture of GMA and TGE.

3. The method of claim 2, wherein GMA has a concentration of 0.05-1% (w/v), and wherein TGE has a concentration of 0.05-1% (w/v).

4. The method of any of claims claim 1-3, wherein the epoxide can be replaced by or combined with an anchoring compound selected from the group comprising a N-acryloxysuccinimide, acrylic acid N-hydroxysuccinimide ester and Acryloxyl-X.

5. The method of any of claims 1-4, wherein molecule-specific labeling comprises immuo-labeling and labeling with other affinity probes.

6. The method of claim 5, wherein immono-labeling comprises incubation with a primary antibody and a fluorophore-conjugated secondary antibody or a (DNA)-barcoded antibody and wherein the other affinity probes comprise fluorophore- or (DNA)-barcoded single-chain variable fragments, nanobodies, ligands, aptamers, affibodies, small molecule binders, target binding peptides and proteins, and oligonucleotide probes.

7. The method of any of the preceding claims, wherein a pan-protein staining is performed at any step of the method after the provision of the tissue sample of interest.

8. The method of any of the preceding claims, wherein the polymerization initiator is APS and/or wherein the polymerization accelerator is TEMED, or wherein the polymerization initiator and the polymerization accelerator are replaced by VA044 or VA050.

9. The method of any of the preceding claims, wherein the tissue sample is neutralized in 0.15-0.5% (w/v) APS and 0.15-0.5% v/v TEMED in water, with 0.15-0.5% (w/v) VA044 or with 0.15-0.5% (w/v) VA050.

10. The method of any of the preceding claims, wherein the tissue sample is a biological sample comprising an organ, a tissue section, an *in vitro* tissue culture, an *in vitro* three-dimensional organ culture, an *in vitro* cell culture or an *in vitro* cultured functional group of cells.

11. The method of any of the preceding claims, wherein the tissue sample is provided as a tissue section having a thickness of around 50-300 µm.

12. The method of any of the preceding claims, wherein the method further comprises analyzing the expanded hydrogel-embedded tissue sample with light microscopy.

13. The method of claim 12 wherein light microscopy comprises bright-field, dark field, wide field, confocal, spinning disc confocal, light sheet and/or line scanning microscopy with signal generation via fluorescence, fluorescence lifetime, harmonic generation, wave mixing, phase contrast, absorption, scattering, polarization, vibrational (Raman) contrast or luminescence.

14. A hydrogel for tissue preservation, traceability of cellular structures and experimental robustness, wherein the hydrogel is prepared according to the method of any of claims 1-13.

15. Use of the hydrogel of claim 14 in light microscopy.
